# EUROPEAN PATENT APPLICATION

(11) **EP 4 696 369 A1**
(43) Date of publication of application: **18.02.2026**
(21) Application number: 24194444.6
(22) Date of filing: 14.08.2024
(51) Int. Cl.: A61N 1/372

(54) **IMPLANTABLE MEDICAL DEVICE AND METHOD FOR OPERATING AN IMPLANTABLE MEDICAL DEVICE**

(71) Applicant: BIOTRONIK SE & Co. KG, 12359 Berlin (DE)
(72) Inventor: VON ARX, Jeffrey A., Lake Oswego, 97035 (US); BROWN, James E., Tigard, 97224 (US); MUESSIG, Dirk, West Linn, 97068 (US)
(74) Representative: Biotronik Corporate Services SE

(57) **Abstract**

An implantable medical device (1) comprises a generator device (10) comprising a processing circuitry (102) for controlling operation of the implantable medical device (1) and a communication circuitry (105) for establishing a communication connection to an external device (2). A housing and a connection block, wherein the connection block is mounted adjacently to the housing, and wherein the generator device is arranged inside the housing. An antenna lead (12), which is connected to and extends from the generator device (10), is operatively connected to the communication circuitry (105) for transmitting communication signals to and/or receiving communication signals from the external device (2). At least a part of the antenna lead is located outside of the connection block and outside of the housing.

## Description

The instant invention concerns an implantable medical device and a method for operating an implantable medical device.

An implantable medical device generally comprises a generator device comprising a processing circuitry for controlling operation of the implantable medical device and a communication circuitry for establishing a communication connection to an external device. The implantable medical device furthermore comprises at least one electrode lead configured for connection to the generator device and comprising an electrode pole arrangement for emitting electrical stimulation signals and/or sensing body signals.

The implantable medical device may for example be a cardiac stimulation device, such as a pacemaker device or a defibrillator device, e.g. an Implantable Pulse Generator (IPG), Implantable Cardioverter-Defibrillator (ICD) or Cardiac Resynchronization Therapy (CRT) device. According to an embodiment, the implantable medical device may be an implantable leadless pacemaker device. In other embodiments, the implantable medical device may for example be a monitoring device for monitoring a body function, as for instance a cardiac loop recorder, or a neuro-stimulation device.

For example within a home monitoring system, the implantable medical device shall communicate with an external device in order to receive e.g. configuration data from the external device or to transmit processing data, for example indicative of an operation of the implantable medical device, to the external device. The external device may serve as a relay for receiving data from and/or transmitting data to a home monitoring service center, at which data may be processed and analyzed, e.g. for assessment by a physician.

Conventionally, dedicated communication techniques are used for establishing a communication between an implantable medical device and an external device, which rests outside of the patient. For such communication techniques, for example an MICS protocol may be employed, using a transmission of (electromagnetic) signals at a frequency around 400 MHz.

At the frequency of the MICS band a communication connection may be established to implanted devices which rest at a substantial implantation depth. There however is a desire to use other communication techniques which are widely available, such as Bluetooth or Bluetooth Low Energy (BLE), allowing for an increased data throughput, but utilizing a frequency range well above the MICS band. By using a standardized communication technique widely used in the field of consumer electronics, it may become possible to establish a connection to an implanted device using a common communication device, such as a smart phone or a tablet computer, hence easing the setup of a home monitoring system.

At increased frequencies, however, a signal dampening due to body tissue increases, such that a communication connection potentially cannot be easily and reliably established to an implanted device at a substantial implantation depth.

In an implantable medical device there may be limitations for the placement of the generator device, making it potentially impossible to adapt an implantation depth in order to allow for a communication using e.g. Bluetooth or Bluetooth Low Energy. For example, the generator device may be implanted left laterally between the musculus serratus anterior and the musculus latissimus dorsi, posing restrictions on a required implantation depth.

US 2020/0197710 A1 discloses methods, systems and devices for wireless signal transmission employing a fractal antenna coupled with a lead. For example, the fractal antenna may be placed, in the context of a deep brain stimulation operation, between a patient's skull and its scalp and may convey signals to the lead.

It is an object of the instant invention to provide an implantable medical device and a method for operating an implantable medical device which allow in an easy and efficient way to establish a communication to an external device.

In one aspect, an implantable medical device comprises: a generator device, comprising: a processing circuitry for controlling operation of the implantable medical device and a communication circuitry for establishing a communication connection to an external device; a housing and a connection block, wherein the connection block is mounted adjacently to the housing, and wherein the generator device is arranged inside the housing; and an antenna lead which is connected to and extends from the generator device, the antenna lead being operatively connected to the communication circuitry for transmitting communication signals to and/or receiving communication signals from the external device, wherein at least a part of the antenna lead is located outside of the connection block and outside of the housing. The described arrangement of the antenna lead ensures an enhanced transmission/receiving signal quality due to a reduction of losses caused by tissue absorption of radio waves.

The implantable medical device comprises a generator device having a communication circuitry, for example a communication chip, which is configured to establish a communication connection to an external device in an RF frequency range, e.g. above 400 MHz, in particular, in a frequency range between 402 and 405 MHz, 800 and 950 MHz, 2 GHz and 3 GHz and/or between 5 GHz and 6 GHz, for example, in a frequency range between 2.4 GHz and 2.5 GHz. Herein, for transmitting signals or for receiving signals within the context of the communication connection, the communication circuitry is connected to a (dedicated) antenna lead enabling signal transmission and/or reception.

As restrictions may exist for implanting the implantable medical device, in particular such that the generator device may, in an implanted state, have to be placed at a substantial implantation depth, an antenna is arranged on an antenna lead which, in an operational state, is connected to the generator device and extends from the generator device.

An antenna lead, in addition to one or multiple electrode leads for performing a therapeutic and/or diagnostic function, hence is used for establishing a communication connection to an external device. Whereas the generator device for example subcutaneously is implanted in a patient, for example at an implantation location between the musculus serratus anterior and the musculus latissimus dorsi, the one or multiple electrode leads extend from the generator device towards a location of interest such that the electrode pole arrangement arranged on the one or multiple electrode leads is placed within the patient at an implantation site to perform a desired therapeutic and/or diagnostic function, e.g. a cardiac stimulation function. In addition to the one or multiple electrode leads, an antenna lead extends from the generator device towards a site to enable a transmission and reception of RF signals to allow for a reliable communication also at increased RF frequencies.

At increased frequencies, signal dampening by body tissue is increased. By using the antenna lead, a radiating structure serving as an antenna is spatially removed from the generator device and is not restricted by the implantation of the generator device. The antenna lead may be placed at an implantation depth which is sufficiently small in order to establish a reliable communication connection at RF frequencies e.g. in a frequency range above 400 MHz.

Generally, the antenna lead may be designed for a dedicated signal transmission and reception in a frequency range above 400 MHz, in particular, in a frequency range between 402 and 405 MHz, 800 and 950 MHz, 2 GHz and 3 GHz and/or between 5 GHz and 6 GHz, for example, in a frequency range between 2.4 GHz and 2.5 GHz.

In one embodiment, the communication circuitry is configured to establish a communication connection to the external device in a frequency range between 402 and 405 MHz , 800 and 950 MHz, 2 GHz and 3 GHz and/or between 5 GHz and 6 GHz. In particular, the communication circuitry may be configured to establish a communication connection to the external device in a frequency range between 2.4 GHz and 2.5 GHz, specifically in the 2.4 GHz ISM spectrum band (2400 to 2483.5 MHz).

In one embodiment, the communication circuitry is configured to establish a communication connection to the external device using Medical Implant Communication Service (MICS), Long Range Wide Area Network (LoRa, LoRaWAN), Bluetooth or Bluetooth Low Energy. For example, the Bluetooth 5.2 standard may be employed.

As another communication technology, for example WiFi may be employed, using a frequency band at 2.4 GHz and/or 5 GHz.

As yet another communication technology, for example 5G may be employed.

In one embodiment, the antenna lead comprises a transmission line section and a radiating section, the transmission line section connecting the radiating section with the generator device. The transmission line section is designed to transmit RF signals between the generator device and the radiating section, such that RF signals may be received at the radiating section and may be transmitted via the transmission line section towards the communication circuitry of the generator device, or vice versa may be transmitted from the communication circuitry towards the radiating section to be radiated towards the external device.

According to an embodiment, at least a part of the radiating section and/or the transmission line section of the antenna lead is located outside of the housing. In an embodiment, at least a part of the radiating section and/or transmission line section of the antenna lead is located outside of the connection block. In an embodiment, at least a part of the radiating section and/or transmission line section of the antenna lead is located outside of the connection block and outside of the housing. In an embodiment, the entire radiating section of the antenna lead is located outside of the connection block and outside of the housing. The described arrangement of the antenna lead ensures an enhanced transmission/receiving signal quality due to a reduction of losses caused by tissue absorption of radio waves.

According to an embodiment, the implantable medical device comprises at least one electrode lead configured for connection to the generator device and comprising an electrode pole arrangement for emitting electrical stimulation signals and/or sensing body signals. According to an embodiment, the antenna lead is separate from at least one electrode lead.

According to an embodiment, the implantable medical device comprises an electrode pole arrangement configured for connection to the generator device for emitting electrical stimulation signals and/or sensing body signals, wherein the electrode pole arrangement does not comprise an elongated lead body. For instance, the electrode pole arrangement is located on the surface of the housing. Alternatively, at least one pole of the electrode pole arrangement is located at a tip portion of the implantable medical device, the tip portion being protruding from the housing or the connection block.

In one embodiment, the transmission line section comprises a ground plane conductor connected to a ground plane member of the generator device and a signal conductor for transporting signals to and/or from the radiating section. The ground plane member of the generator device may be formed for example by a housing of the generator device. The ground plane conductor of the transmission line section is connected to the ground plane member and hence is on a common ground potential with the ground plane member of the generator device. The signal conductor, together with the ground plane conductor, forms a shielded transmission line which enables an RF signal transmission beneficially at minimal loss between the radiating section and the communication circuitry of the generator device.

In one embodiment, the ground plane conductor and the signal conductor are coaxially arranged with respect to one another. The ground plane conductor and the signal conductor thus form a coaxial transmission line, wherein the signal conductor forms an inner conductor of the transmission line and the ground plane conductor provides for a surrounding shielding. The ground plane conductor and the signal conductor herein are insulated from one another by an insulation layer.

For example, the ground plane conductor may be formed by a helically wound wire, a metal mesh or a laser-cut hypotube (a tube which is laser cut to provide a certain degree of flexibility on the tube, similar to a stent-like structure). The ground plane conductor may for example be made from a biocompatible metal material, for example MP35N, gold, platinum, platinum-iridium, DFT (drawn filled tubing) such as MP35N tubing with silver or tantalum core, or some other suitable biocompatible conductor.

The signal conductor for example is coaxially arranged with respect to the ground plane conductor and is formed e.g. by a straight wire, a cable, or a helically coiled wire extending along the central axis of the transmission line section. The signal conductor may for example be formed by a biocompatible material, for example MP35N, gold, platinum, platinum-iridium, DFT (drawn filled tubing) such as MP35N tubing with silver or tantalum core, or some other suitable biocompatible conductor.

An insulation layer in between the signal conductor and the ground plane conductor may for example be formed by silicon, polyurethane, or some other suitable biocompatible insulator material.

In other embodiments, the ground plane conductor and the signal conductor are arranged to form a twisted pair of cables formed by insulated wires.

In yet other embodiments, the ground plane conductor and the signal conductor are formed by different layers of a layered structure, for example thin-film metal layers printed on a flexible LCP or polyimide substrate. For example, the transmission line section, comprising the signal conductor and the ground plane conductor, may be formed by a strip line or microstrip line. For example, a layered structure forming the transmission line section, for example a thin-film transmission line substrate, is flexible and may be coiled or twisted to allow flexibility of the transmission line section along different spatial directions.

In one embodiment, the transmission line section comprises a ground plane electrode connected to the ground plane conductor for electrically coupling to tissue. The ground plane conductor is electrically connected, at a proximal end, to the ground plane member of the generator device, for example to the housing of the generator device. The ground plane electrode is spatially removed from the ground plane member of the generator device and for example is arranged at a distal end of the transmission line section distant from the generator device. By means of the ground plane electrode an electrical coupling to surrounding tissue may be established, such that the distal end of the ground plane conductor of the transmission line section is electrically coupled to the electrical ground potential of the surrounding tissue, corresponding to the ground potential of the ground plane member, for example the housing, of the generator device.

In one embodiment, the radiating section comprises a radiating element extending from the transmission line section. The radiating element is not shielded by an associated ground plane conductor and hence extends from the transmission line section in order to protrude from the ground plane conductor of the transmission line section.

The radiating element may for example be formed by a straight wire. In other embodiments, the radiating element may be formed by a helically wound wire or a meandering cable conductor. The radiating element is for example made from a biocompatible metal material such as MP35N, gold, platinum, platinum iridium, or DFT (drawn filled tubing) such as MP35N tubing with silver or tantalum core.

In yet other embodiments the radiating element is made of a patterned thin-film metal trace on a flexible biocompatible dielectric material such as a gold trace on a flexible polyimide or LCP substrate. In one embodiment, a thin-film radiating element formed in this manner is coiled or twisted to allow for a flexibility in different spatial directions.

In one embodiment, the radiating section comprises a first insulation member enclosing the radiating element and electrically insulating the radiating element. The insulation member insulates the radiating element from surrounding body tissue and protects the radiating element from bodily fluids. The insulation member may for example be formed from a polyurethane or a silicon material or a combination thereof.

In addition, the radiating element may comprise an insulating coating for example made of ETFE, PFA, FEP, PTFE, or other biocompatible insulating material for increased protection against insulation damage due to, for example, wear and fatigue.

In one embodiment, the transmission line section comprises a second insulation member. The first insulation member and the second insulation member are, in one embodiment, isodiametric, such that the first insulation member and the second insulation member comprise the same outer diameter. The second insulation member of the transmission line section may for example be formed from the same material as the first insulation member of the radiating section. For example, the second insulation member is formed from polyurethane or a silicon material or a combination thereof. By means of the second insulation member in particular the ground plane conductor of the transmission line section may be electrically insulated towards surrounding tissue and may be protected from bodily fluids.

In one embodiment, the transmission line section has a length in between 3 cm to 20 cm. The length of the transmission line section may be chosen dependent on the implantation location of the generator device, such that the antenna lead may extend from the generator device, in an implanted state, towards a location at which the radiating section of the antenna lead may be placed at shallow depth beneath the patient's skin to enable an efficient RF communication with minimal tissue signal loss.

In one embodiment, the radiating section has a length in between 0.1 cm to 2 centimeters, for example between 0.1 cm and 1.2 cm. The length of the radiating section may be chosen dependent on the frequency which shall be used for communication, the length of the radiating section for example relating to the wavelength of transmission signals, for example corresponding to a quarter wavelength at a center frequency of a desired frequency range, wherein the dielectric constant of surrounding tissue and e.g. an insulation member should be taken into account in order to properly match the length of the radiating section to the desired frequency range.

In one embodiment, the antenna lead comprises a stylet opening for inserting a stylet. By means of the stylet, the antenna lead may be actively guided for example along an implantation channel by stiffening the antenna lead during implantation. The stylet opening for example may be formed within the antenna lead such that it extends eccentrically along the antenna lead.

One or multiple stylet openings may be provided.

During implantation a stylet is introduced into the stylet opening within the antenna lead in order to actively guide the antenna lead along an implantation channel. Upon completion of the implantation the stylet is removed and discarded.

In one embodiment, the antenna lead comprises a fixation member for anchoring the antenna lead with respect to the at least one electrode lead and/or with respect to tissue.

The antenna lead generally forms a separate lead in comparison to the at least one electrode lead. In order to provide for a fixation of the antenna lead in the patient in an implanted state of the implantable medical device, the antenna lead however may be anchored on an electrode lead, such that the antenna lead does not need to be fixated separately from the at least one electrode lead within the patient during implantation, hence easing the implantation procedure.

For example, the fixation member may be formed by a tube element for receiving the at least one electrode lead therewithin. For implantation, the fixation member may be placed on an already implanted electrode lead and may be guided along the electrode lead, such that the antenna lead is implanted to extend along the associated electrode lead.

The fixation member may for example be formed by a thin-walled tube, having a length for example in between 0.5 cm to 1.2 cm, and is for example bonded to the antenna lead, for example to the transmission line section. The fixation member may be made from a silicon or polyurethane material. The fixation member may for example be arranged on or close to the distal end of the transmission line section. One or multiple fixation members may be used in order to provide for a fixation to an associated electrode lead along the length of the antenna lead.

In other embodiments, the fixation member may be formed by a spiral cuff feature. A spiral cuff feature of this kind may for example be formed by an arrangement of thin sheets of silicon bonded together, one stretched prior to bonding. The tensioning of the stretched sheet causes the bonded sheets to curl into a spiral form. The spiral cuff may be straightened, for example using tweezers, and when released will again curl up into its spiral form. An anchoring to an associated electrode lead in this case may be achieved in that the antenna lead is placed next to the electrode lead and the spiral cuff is straightened out and then released in order to curl around the electrode lead.

In other embodiments, the antenna lead may be sutured to tissue and hence may be anchored independent of the one or the multiple electrode leads.

In yet other embodiments, an anchoring structure may be formed by a circular or paddle-shaped feature at the distal end of the antenna lead, which may provide for an anchoring on tissue. The radiating element of the antenna lead herein may extend into the anchoring structure.

The antenna lead, in one embodiment, is fixedly connected to the generator device and hence is intended to not be released from the generator device. For implantation of the implantable medical device, the antenna lead for example is implanted together with the generator device, wherein the one or multiple electrode leads may be implanted at a prior to implantation step, hence allowing to anchor the antenna lead to an already implanted electrode lead.

By fixedly connecting the antenna lead to the generator device, an optimized RF connection between the antenna lead and the generator device may be established, in particular providing for an impedance matching in between the transmission line section and the communication circuitry of the generator device.

The electrode pole arrangement of the at least one electrode lead for example comprises multiple electrode poles. One or multiple electrode poles of the electrode pole arrangement may be placed on one or multiple electrode leads. In addition, one or multiple electrode poles may be formed by a housing of the generator device.

In another aspect, a method for operating an implantable medical device comprises: providing a generator device comprising a processing circuitry for controlling operation of the implantable medical device and a communication circuitry for establishing a communication connection to an external device; providing a housing and a connection block, wherein the connection block is mounted adjacently to the housing, and wherein the generator device is arranged inside the housing; and transmitting communication signals to and/or receiving communication signals from the external device using an antenna lead, which is connected to and extends from the generator device, wherein at least a part of the antenna lead is located outside of the connection block and outside of the housing, the antenna lead being operatively connected to the communication circuitry.

The advantages and advantageous embodiments described above for the implantable medical device equally apply also to the method, such that it shall be referred to the above in this respect.

The various features and advantages of the present invention may be more readily under-stood with reference to the following detailed description and the embodiments shown in the drawings. Herein,
- Fig. 1: shows a schematic drawing of an implantable medical device in an implanted state in a patient;
- Fig. 2: shows the implantable medical device of Fig. 1, illustrating signal vectors spanned by electrode poles of an electrode pole arrangement of the device;
- Fig. 3: shows a view of an embodiment of a generator device and an antenna lead fixedly connected to the generator device;
- Fig. 4: shows another view of the arrangement of Fig. 3;
- Fig. 5: shows yet another view of the arrangement of Fig. 3;
- Fig. 6: shows a view of another embodiment of a generator device and an antenna lead connected to the generator device;
- Fig. 7: shows a view of an embodiment of a generator device and an antenna lead connected to the generator device;
- Fig. 8: shows an enlarged view in the region X of Fig. 7;
- Fig. 9: shows a schematic view of an antenna lead connected to a generator device;
- Fig. 10: shows a schematic cross-section along line I-I of Fig. 9; and
- Fig. 11: shows a schematic cross-section along line II-II of Fig. 9.

Subsequently, embodiments of the invention shall be described in detail with reference to the drawings. In the drawings, like reference numerals designate like structural elements.

It is to be noted that the embodiments are not limiting for the invention, but merely represent illustrative examples.

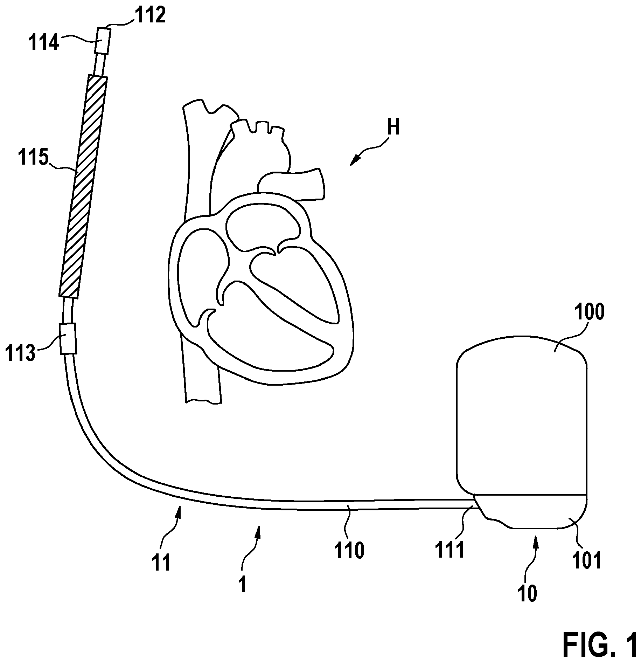

Referring to Fig. 1, in a setup of a therapeutic and/or diagnostic system an implantable medical device 1 is for example designed as a non-transvenous implantable medical device 1. The non-transvenous implantable medical device 1 is implanted such that the implantable medical device 1 is completely external to the heart H, the implantable medical device 1 comprising a generator device 10 encapsulated within a housing 100, and an electrode lead 11 connected to a connection block 101 of the generator device 10 at a proximal end 111 and carrying electrode poles 113, 114 as well as a shock electrode 115 in the shape of a coil formed on a distal portion close to a distal end 112 of the electrode lead 11. The electrode poles 113, 114, for example formed as ring electrodes on either side of the shock electrode 115, serve to sense cardiac signals for processing within the generator device 10 of the implantable medical device 1, such that based on sensed signals an arrhythmia may be identified and a shock pulse may be generated for providing for a defibrillation therapy.

The implantable medical device 1, in the embodiment of Fig. 1, is designed for a non-transvenous implantation, that is an implantation external to the patient's heart H. In particular, the electrode lead 11 connected to the generator device 10 e.g. shall rest outside of the patient's heart H and shall not extend transvenously into the heart, the shock electrode 115 hence, in an implanted state, being placed outside of the heart H for providing for a defibrillation therapy.

For example, the generator device 10 may be implanted subcutaneously in a patient. The electrode lead 11, with a lead body 110, may extend from the generator device 10 towards the sternum of the patient, the electrode lead 11 for example tunneling through tissue in the region of the sternum and being placed beneath the sternum of the patient.

In other embodiments, the implantable medical device 1 may be designed for a transvenous implantation, that is an implantation using one or multiple electrode leads to be implanted in the patient's heart H.

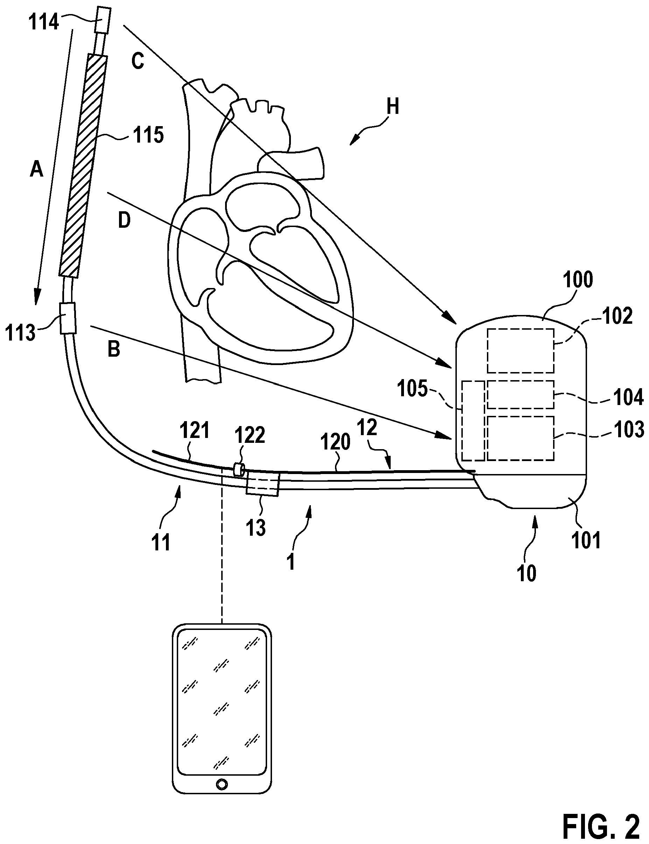

Referring now to Fig. 2, the generator device 10 generally comprises a processing circuitry 102 for controlling operation of the implantable medical device 1. In addition, the generator device 10 comprises a pulse generation circuitry 103 and an energy storage 104, in particular in the shape of a battery.

The processing circuitry 102 in particular may serve to process signals sensed via an electrode pole arrangement formed by the electrode poles 113, 114 arranged on the electrode lead 11 and additional poles, such as a shock electrode 115 and the housing 100 of the generator device 10. The different poles of the electrode pole arrangement may form a pair of electrode poles in between which signal vectors A, B, C, D are spanned, as illustrated in Fig. 2, the different signal vectors A, B, C, D allowing to sense electrocardiogram signals from the patient's heart H with a different spatial sensitivity, hence allowing to sense and process information from the patient's heart H in a multichannel processing.

Alternatively or in addition, stimulation signals may be output across one or multiple of the signal vectors A, B, C, D.

An implantable medical device 1, for example as shown in Figs. 1 and 2, may be configured to perform a defibrillation therapy. In other embodiments, the implantable medical device 1 may be configured for a cardiac pacing function, to perform a monitoring function, or to perform a neuromodulation function.

As illustrated in Fig. 2, the implantable medical device 1 comprises a communication circuitry 105 for establishing a communication connection with an external device 2, for example within a home-monitoring system.

The external device 2 may for example be a consumer communication device, such as a smart phone or a tablet computer. Both the communication circuitry 105 and a communication circuitry of the external device 2 herein may be configured to set up a communication connection according to a standardized communication protocol, for example employing Bluetooth, Bluetooth Low Energy or WiFi.

The implantable medical device 1 shall be enabled to establish a communication with the external device 2 in an RF frequency range e.g. above 2 GHz, for example in the 2.4 GHz ISM band used by Bluetooth or Bluetooth Low Energy. In that a communication takes place by using a standardized communication technology as employed by and as implemented generally on consumer communication devices, a communication connection between the implantable medical device 1 and the external device 2 may be established using a widely available communication technique, hence reducing the requirements for an adaptation of the external device 2 for communicating with the implantable medical device 1.

For establishing a communication, the communication circuitry 105 of the generator device 10, for example a communication chip set for Bluetooth or Bluetooth Low Energy communication, a WiFi communication or a 5G communication, is connected to an antenna lead 12. The antenna lead 12 is fixedly connected to the generator device 10 such that it is generally not intended to be released from the generator device 10.

The antenna lead 12 comprises a transmission line section 120 and a radiating section 121 connected by the transmission line section 120 to the generator device 10 such that the radiating section 121 is in operative connection with the communication circuitry 105 of the generator device 10.

By using the antenna lead 12, communication capabilities of the implantable medical device 1 become largely independent of the implantation site of the generator device 10. In particular, the generator device 10 of the implantable medical device 1 may be implanted by placing it e.g. in a pocket left laterally in between the musculus serratus anterior and the musculus latissimus dorsi, posing restrictions on a required implantation depth of the generator device 10. The antenna lead 12 however may be implanted to reach towards an implantation site at which an improved RF communication may be enabled.

The implantable medical device 1 comprises a hermetically sealed housing 100, wherein generator device 10 is arranged inside the housing 100. For instance, according to an embodiment, the implantable device 1 comprises a connection block 101, wherein connection block 101 is mounted adjacently to housing 100. Moreover, according to an embodiment, at least a part of antenna lead 12 is located outside of housing 100. The described arrangement of antenna lead 12 ensures an enhanced transmission/receiving signal quality due to a reduction of losses caused by tissue absorption of radio waves.

At least a part of the radiating section 121 and/or the transmission line section 120 of the antenna lead 12 may be located outside of housing 100. In an embodiment, at least a part of the radiating section 121 and/or transmission line section 120 of the antenna lead 12 is located outside of connection block 101. In an embodiment, at least a part of the radiating section 121 and/or transmission line section 120 of the antenna lead 12 is located outside of connection block 101 and outside of housing 100. In an embodiment, the entirety of radiating section 121 of the antenna lead 12 is located outside of connection block 101 and outside of housing 100. The described arrangement of the antenna lead 12 ensures an enhanced transmission/receiving signal quality due to a reduction of losses caused by tissue absorption of radio waves.

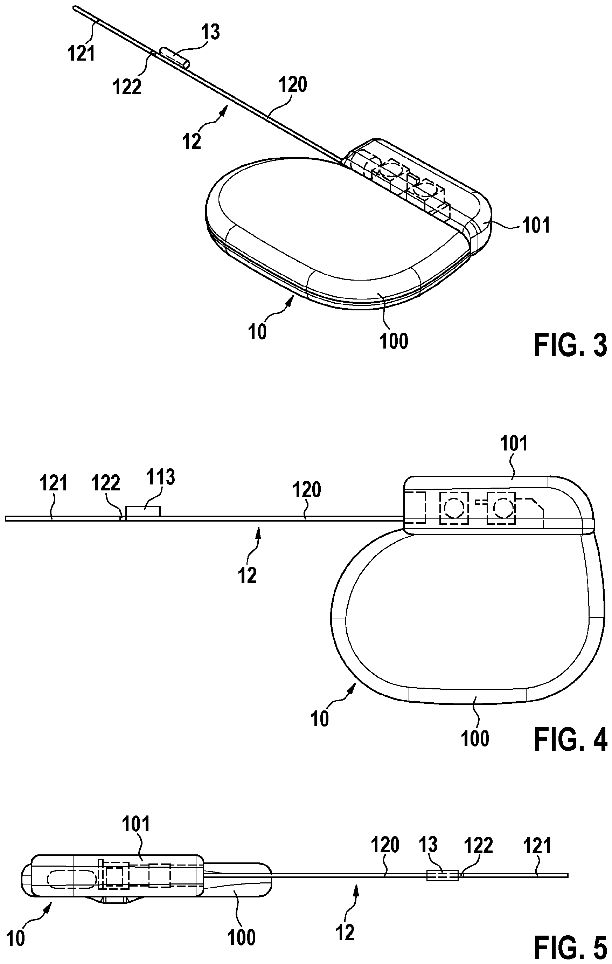

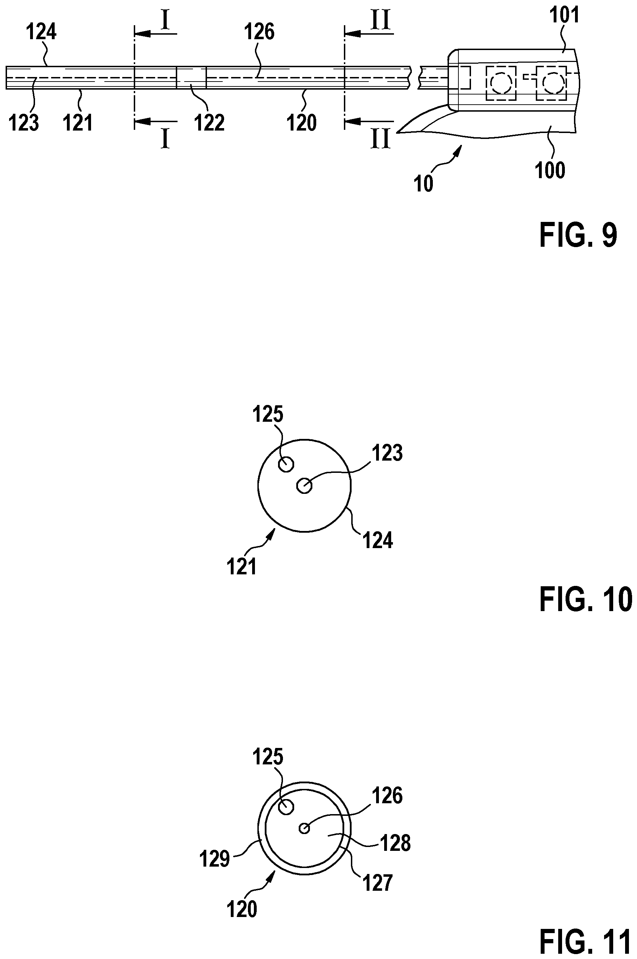

Referring now to Figs. 3 to 5 and Figs. 9 to 11, the transmission line section 120 of the antenna lead 12, in one embodiment, is formed by a coaxial structure having a ground plane conductor 127 and a signal conductor 126 arranged coaxially within the ground plane conductor 127, as this is illustrated in Fig. 11. The ground plane conductor 127 provides for a shielding of the transmission line section 120 and is operatively connected to a ground plane member of the generator device 10, for example to the housing 100 of the generator device 10, such that the ground plane conductor 127 is on the same ground potential as the ground plane member of the generator device 10. The signal conductor 126 extends within the ground plane conductor 127 along the length of the transmission line section 120 and is electrically insulated from the ground plane conductor 127 by an insulation layer 128, as visible from Fig. 11.

At a distal end the transmission line section 120 comprises a ground plane electrode 122, formed for example by a ring electrode extending circumferentially about the transmission line section 120, configured for establishing an electrical coupling to surrounding tissue. The ground plane electrode 122 is electrically coupled to the ground plane conductor 127 and hence causes the ground plane conductor 127 to be on the ground potential of the surrounding tissue in the vicinity of the distal end of the transmission line section 120.

Distally with respect to the transmission line section 120 the radiating section 121 protrudes from the transmission line section 120. The radiating section 121 comprises a radiating element 123 electrically connected to the signal conductor 126 of the transmission line section 120. The radiating element 123 is electrically insulated towards the outside by means of an insulation member 124 enclosing the radiating element 123.

The transmission line section 120 beneficially is electrically insulated by means of an insulation member 129 surrounding and enclosing the ground plane conductor 127, as visible from Fig. 11. The insulation member 129 of the transmission line section 120 and the insulation member 124 of the radiating section 121 are, in one embodiment, isodiametric with respect to one another. Beneficially, the ground plane electrode 122 also is isodiametric with the insulation members 124, 129.

The radiating element 123 for example is formed by a straight wire or straight cable. For example, the radiating element 123 is formed from a wire or cable of biocompatible metal material such as MP35N, gold, platinum, platinum iridium, or DFT (drawn filled tubing) such as MP35N tubing with silver or tantalum core.

The signal conductor 126 for example is formed from a wire or cable longitudinally extending along the transmission line section 120 coaxially with respect to the ground plane conductor 127. The signal conductor 126 for example is made from a wire or cable of biocompatible metal material such as MP35N, gold, platinum, platinum iridium, or DFT (drawn filled tubing) such as MP35N tubing with silver or tantalum core.

For example, the radiating element 123 and the signal conductor 126 are formed in one piece by a single, continuous wire or cable.

The insulation members 124, 129 for example are made from an electrically insulating polyurethane or a silicon material or a combination thereof.

The ground plane conductor 127 for example is formed by a coiled wire, a mesh or a laser-cut hypotube. The ground plane conductor 127 may be fabricated from a similar material as the signal conductor 126.

The transmission line section 120 may for example have a length between 3 cm to 20 cm. The length of the transmission line section 120 in particular may be chosen dependent on a desired placement of the radiating section 121 in view of an implantation site of the generator device 10. The transmission line section 120 in particular should be long enough to place the radiating section 121 at a shallow implantation depth at a location allowing for a beneficial RF communication, for example unhindered by a patient's arm movement.

The radiating section 121 may have a length in between 0.1 cm to 2 cm, for example between 0.1 cm and 1.2 cm. The length of the radiating section 121, in particular the length of the radiating element 123 of the radiating section 121, should depend on a desired communication frequency and is chosen dependent on the corresponding wavelength, taken the dielectric properties in the vicinity of the antenna lead 12 into account. For example, the length of the radiating element 123 may be chosen such that it corresponds (roughly) to a quarter wavelength at a desired communication frequency, taking the dielectric constant of surrounding tissue as well as of the insulation member 124 into account.

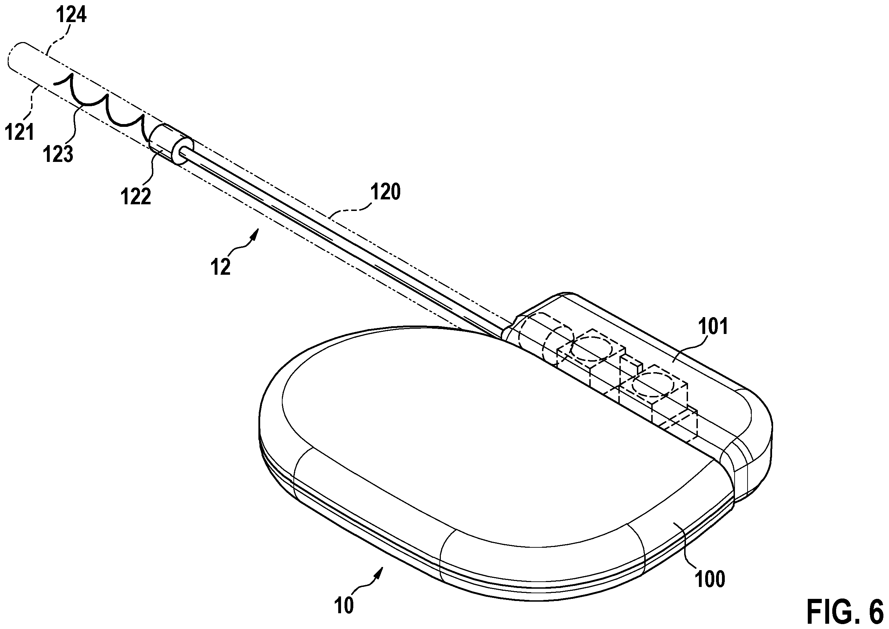

Referring now to Fig. 6, in other embodiments the radiating element 123 may have the shape of a helically wound wire, as illustrated in Fig. 6.

Other structures for the radiating element 123 are conceivable. For example, the radiating element 123 may be formed by a meandering cable conductor or by a patterned thin film metal trace on a flexible biocompatible dielectric material such as a gold trace on a flexible polyimde or LCP substrate.

Likewise, the transmission line section 120 may be formed by different structures, such as thin film metal layers printed on a flexible polyimde or LCP substrate.

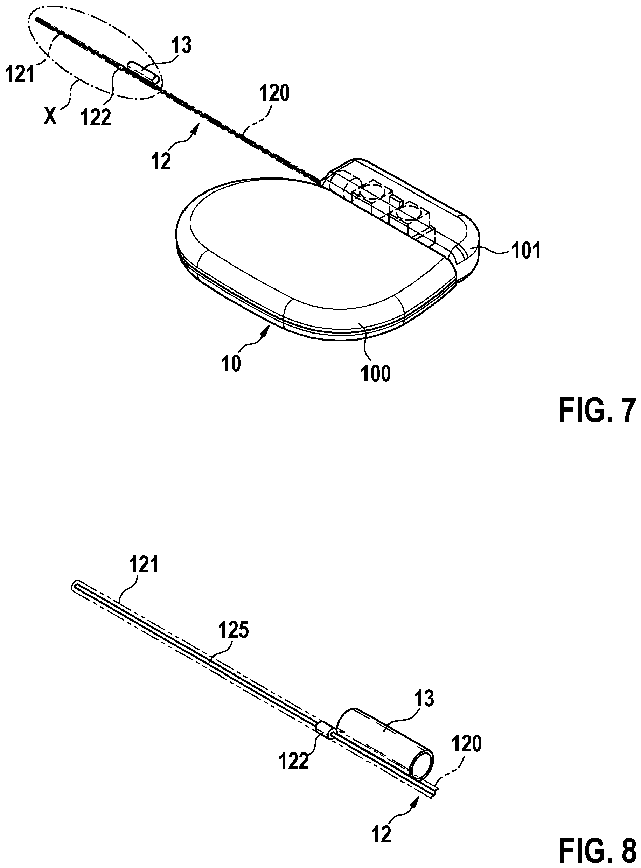

Referring now to Figs. 7 and 8 in view of Figs. 9 to 11, the antenna lead 12 may comprise a stylet opening 125 extending longitudinally along the length of the antenna lead 12 and allowing for an insertion of a stylet for implantation of the antenna lead 12 along an implantation channel. By using a stylet the antenna lead 12 may be stiffened for implantation, such that the antenna lead 12 may be actively guided along an implantation channel to suitably implant the antenna lead 12 within a patient.

As visible from Figs. 3 to 5, in one embodiment the antenna lead 12 comprises a fixation member 13 arranged for example at a distal location on the transmission line section 120, the fixation member 13 being configured for providing for an anchoring for example to an electrode lead 11 of the implantable medical device 1.

The fixation member 13 may for example be formed by a tube element which is configured for receiving an electrode lead 11 within. The fixation member 13 for example is formed from a silicon material and is arranged on the antenna lead 12 at a location distant from the generator device 10.

For example, multiple fixation members 13 may be used on the antenna lead 12 to provide for a suitable anchoring of the antenna lead 12 in an implanted state.

For implantation, for example an arrangement of electrode leads 11 is implanted in an initial implantation step, upon which the generator device 10 with the antenna lead 12 fixedly connected to the generator device 10 is implanted. For this, the fixation member 13 is placed on the already implanted electrode lead 11 and is caused to slide along the electrode lead 11, such that the antenna lead 12 is introduced along the implantation channel of the electrode lead 11 and, once it reaches its final position, is anchored to the electrode lead 11 by means of the fixation member 13.

Other fixation means or conceivable. For example a fixation member may be formed by a spiral cuff. In other embodiments a fixation of the antenna lead 12 in an implanted state may be provided independently of an electrode lead 11 for example by suturing the antenna lead 12 to tissue. In yet other embodiments, an anchoring structure such as a circular or paddle-like structure may be provided on the antenna lead 12 in order to provide for an anchoring on tissue.

The electrode lead 11 is formed flexible such that it may flexibly be arranged and implanted within the patient. The electrode lead 11 in particular is flexibly bendable. A lead body 110 for example may be formed from a silicon material. The antenna lead 12 beneficially is enclosed within the material of the lead body 110.

Likewise, the antenna lead 12 beneficially is flexibly bendable to follow an implantation channel.

In one embodiment the electrode lead 11, at its proximal end 111, comprises a connector having a multiplicity of contact elements, for example according to the definition of an IS4/DS4 connector. The connector of the electrode lead 11 may be plugged into a corresponding connector in the shape of a plug receptacle formed on a connection block 101 of the generator device 10.

In contrast, the antenna lead 12 beneficially is fixedly connected to the generator device 10, i.e. in a non-releasable fashion without the use of connectors, for example via a feedthrough in the housing 100 of the generator device 10.

### List of reference numerals

- 1: Non-transvenous implantable medical device
- 10: Generator device
- 100: Housing / Ground plane member
- 101: Connection block
- 102: Processing circuitry
- 103: Pulse generation circuitry
- 104: Energy storage (battery)
- 105: Communication circuitry
- 11: Electrode lead
- 110: Lead body
- 111: Proximal end
- 112: Distal end
- 113: Electrode pole
- 114: Electrode pole
- 115: Shock electrode (coil)
- 12: Antenna lead
- 120: Transmission line section
- 121: Radiating section
- 122: Ground plane electrode
- 123: Radiating element
- 124: Insulation member
- 125: Stylet opening
- 126: Signal conductor
- 127: Ground plane conductor
- 128: Insulation layer
- 129: Insulation member
- 13: Fixation member
- 2: External device
- A-D: Reception vector
- H: Heart

## Claims

1. An implantable medical device (1), comprising:
a generator device (10) comprising a processing circuitry (102) for controlling operation of the implantable medical device (1) and a communication circuitry (105) for establishing a communication connection to an external device (2),
a housing (100) and a connection block (101), wherein the connection block (101) is mounted adjacently to the housing (100), and wherein the generator device (10) is arranged inside the housing (100),
an antenna lead (12), which is connected to and extends from the generator device (10), the antenna lead (12) being operatively connected to the communication circuitry (105) for transmitting communication signals to and/or receiving communication signals from the external device (2),
wherein at least a part of the antenna lead (12) is located outside of the connection block (101) and outside of the housing (100).

2. The implantable medical device (1) according to claim 1, wherein the antenna lead (12) is configured to transmit communication signals to and/or receive communication signals from the external device (2) in a frequency range above 400 MHz, in particular, in a frequency range between 402 and 405 MHz, 800 and 950 MHz, 2 GHz and 3 GHz and/or between 5 GHz and 6 GHz, for example, in a frequency range between 2.4 GHz and 2.5 GHz.

3. The implantable medical device (1) according to claim 1 or 2, wherein the communication circuitry (105) is configured to establish a communication connection to the external device (2) using MICS, LoRa, Bluetooth, Bluetooth Low Energy, WiFi, or 5G.

4. The implantable medical device (1) according to one of claims 1 to 3, wherein the antenna lead (12) comprises a transmission line section (120) and a radiating section (121), the transmission line section (120) connecting the radiating section (121) with the generator device (10), wherein at least a part of the radiating section (121) and/or transmission line section (120) of the antenna lead (12) is located outside of the connection block (101) and outside of the housing (100).

5. The implantable medical device (1) according to one of the preceding claims, wherein the implantable medical device comprises at least one electrode lead (11) configured for connection to the generator device (10) and comprising an electrode pole arrangement (113-115) for emitting electrical stimulation signals and/or sensing body signals; wherein the antenna lead (12) is separate from the electrode lead (11).

6. The implantable medical device (1) according to claim 4 or 5, wherein the transmission line section (120) comprises a ground plane conductor (127) connected to a ground plane member (100) of the generator device (10) and a signal conductor (126) for transporting signals to and/or from the radiating section (121).

7. The implantable medical device (1) according to claim 6, wherein the ground plane conductor (127) and the signal conductor (126) are coaxially arranged with respect to one another, or are arranged to form a twisted pair, or are formed by different layers of a layered structure.

8. The implantable medical device (1) according to claim 6 or 7, wherein the transmission line section (120) comprises a ground plane electrode (122) connected to the ground plane conductor (127) for electrically coupling to tissue.

9. The implantable medical device (1) according to claim 8, wherein the transmission line section (120) comprises a distal end distant from the generator device (10), the ground plane electrode (122) being arranged at the distal end of the transmission line section (120).

10. The implantable medical device (1) according to one of claims 4 to 9, wherein the radiating section (121) comprises a radiating element (123) extending from the transmission line section (120).

11. The implantable medical device (1) according to claim 10, wherein the radiating section (121) comprises a first insulation member (124) enclosing the radiating element (123) and electrically insulating the radiating element (123).

12. The implantable medical device (1) according to one of the preceding claims, wherein the antenna lead (12) comprises a stylet opening (125) for inserting a stylet.

13. The implantable medical device (1) according to one of the preceding claims, wherein the antenna lead (12) comprises a fixation member (13) for anchoring the antenna lead (12) with respect to the at least one electrode lead (11) and/or with respect to tissue.

14. The implantable medical device (1) according to claim 13, wherein the fixation member (13) is formed by a tube element for receiving the at least one electrode lead (11) therewithin.

15. A method for operating an implantable medical device (1), the method comprising:
providing a generator device (10) comprising a processing circuitry (102) for controlling operation of the implantable medical device (1) and a communication circuitry (105) for establishing a communication connection to an external device (2);
providing a housing (100) and a connection block (101), wherein the connection block (101) is mounted adjacently to the housing (100), and wherein the generator device (10) is arranged inside the housing (100),
transmitting communication signals to and/or receiving communication signals from the external device (2) using an antenna lead (12), which is connected to and extends from the generator device (10), wherein at least a part of the antenna lead (12) is located outside of the connection block (101) and outside of the housing (100), the antenna lead (12) being operatively connected to the communication circuitry (105).
